# EUROPÄISCHE PATENTSCHRIFT

(11) **EP 2 914 895 B1**
(45) Veröffentlichungstag und Bekanntmachung des Hinweises auf die Patenterteilung: **19.08.2020**
(21) Anmeldenummer: 12781273.3
(22) Anmeldetag: 04.09.2012
(51) Int. Cl.: F17D 1/17, F17D 1/16, F17D 1/18, F17D 3/12, C07C 29/151, C10G 71/00, C10G 3/00

(54) **VERFAHREN ZUR VERBESSERUNG DER TRANSPORTFÄHIGKEIT VON SCHWEREM ROHÖL**
METHOD FOR IMPROVING THE TRANSPORTABILITY OF HEAVY CRUDE OIL
PROCÉDÉ D'AMÉLIORATION DE L'APTITUDE À L'ÉCOULEMENT DE PÉTROLE BRUT LOURD

(43) Veröffentlichungstag der Anmeldung: 09.09.2015
(73) Patentinhaber: GasConTec GmbH, 61348 Bad Homburg v. d. Höhe (DE)
(72) Erfinder: Wagner, Ulrich, 06406 Bernburg OT Biendorf (DE); Balthasar, Wolff, 40833 Ratingen (DE)
(74) Vertreter: Söylemezoglu, Mustafa Nazim
(86) Internationale Anmeldenummer: PCT/DE2012/100262
(87) Internationale Veröffentlichungsnummer: WO 2014/036982

(56) Entgegenhaltungen:
- WO-A1-03/020636
- WO-A2-2009/012154
- DE-A1- 2 451 342
- US-A- 4 027 688
- US-B2- 7 861 737

## Beschreibung

Die Erfindung bezieht sich auf ein Verfahren zur Verbesserung der Transportfähigkeit von schwerem Rohöl, wobei die Viskosität des schweren Rohöls mittels eines Zusatzes verringert werden soll.

Es ist allgemein bekannt, in einer Erdöllagerstätte gefördertes Erdöl als Rohöl zur weiteren Verarbeitung in einer Raffinerie über Rohrleitungen zu transportieren, wobei sich diese über Entfernungen von mehreren tausend Kilometern erstrecken können.

Lassen sich in einer Erdöllagerstätte nur noch schwere oder Superschwere Rohöle gewinnen, also solche z.B. mit einer Viskosität von in etwa 40 000 mPa s bzw. 24° API (API = amerikanische Dichteeinheit für Rohöl), so ist ein längerer Transport derartiger Rohöle durch Rohrleitungen nicht mehr möglich bzw. unwirtschaftlich.

Es wurde daher bereits nach verschiedenen Möglichkeiten gesucht, die Transportfähigkeit von schwerem Rohöl, insbesondere durch Verringerung der Viskosität, zu verbessern. Aus der DE 36 09 641 A1 ist bekannt, zum Transport von zähfließendem Rohöl dieses in eine Öl-in-Wasser-Emulsion mit mindestens 10 bis 15 % Wasser unter Zusatz eines speziellen Emulgators auf Basis Oxethylat umzuwandeln.

Gemäß WO 2011/006024 A2 wird zur Verringerung der Viskosität vorgeschlagen, ein Polymer bestehend aus einem nicht-ionischen Monomeren und mindestens 25 Molprozent kationischer Monomere einzusetzen.

Der Zusatz von Emulgatoren oder Polymeren als Verdünnungsmittel ist mit zusätzlichen Kosten verbunden und erfordert, dass diese vor der Raffination des Rohöls wieder entfernt werden müssen.

In der DE 2 039 329 A wird zur Transportverbesserung vorgeschlagen, Rohöl auf Temperaturen von 340 bis 650 °C zu erhitzen. Dies ist jedoch mit einem erheblichen Aufwand verbunden und bei Transportstrecken von mehreren tausend Kilometern wirtschaftlich nicht realisierbar.

Die DE 24 51 342 A1 aus dem Stand der Technik beschreibt ein Verfahren zum Ferntransport fossiler Energieträger, wobei ein Teil eines fossilen Primärenergieträgers vor dem Transport in Methanol umgesetzt und anschließend dem zu transportierenden Energieträger zugesetzt wird.

Die US 4 027 688 A aus dem Stand der Technik beschreibt, dass fossile Brennstoffe wie Rohöl oder Kohle durch ein Rohr transportiert werden, indem einige der Bestandteile des fossilen Brennstoffs wie Erdgas in Methanol umgewandelt werden. Das Methanol wird dann verwendet, mit mit dem fossilen Brennstoff eine Emulsion zu bilden. Die Emulsion wird per Pipeline transportiert.

Die US 7 861 737 B2 aus dem Stand der Technik, von welcher die vorliegende Erfindung als nächstkommend ausgeht, beschreibt ein Optimierungsverfahren für den Transport von schwerem Rohöl, bei dem dem Rohöl mindestens ein Lösungsmittel zugesetzt wird. Gemäß dem Verfahren wird eine vorbestimmte Menge Dimethylether (DME) unter Druck zugegeben, um die Viskosität des Rohöls einzustellen.

Aufgabe der Erfindung ist es, ein Verfahren zur Verbesserung der Transportfähigkeit von schwerem Rohöl zu schaffen, mittels Zusätzen, die zu einer Qualitätsverbesserung des Rohöls führen und keine nachteiligen Auswirkungen auf die Weiterverarbeitung des Rohöls haben.

Erfindungsgemäß wird die Aufgabe durch die im Anspruch 1 angegebenen Merkmale gelöst. Vorteilhafte Ausgestaltungen und Weiterbildungen der Verfahrensweise sind Gegenstand der Ansprüche 2 bis 13.

Aus dem bei der Gewinnung von schwerem Rohöl in Erdöllagerstätten anfallendem Erdgas und/oder Erdölbegleitgas, z.B. bei einer sogenannten Cluster-Förderung, wird das Begleitgas mittels einer Fluidabtrenneinrichtung abgetrennt. Das Erd- bzw. Begleitgas wird nunmehr nicht mehr, wie ansonsten üblich, zurückverpresst oder abgefackelt, sondern einer wirtschaftlichen Verwertung zugeführt.

Dieses Gas wird in mehreren getrennten Stufen zuerst in ein Methanol-Wasser-Gemisch umgewandelt, das auf einen Wassergehalt von vorzugsweise 4 % eingestellt wird. Im nachfolgenden Verfahrensschritt wird der Prozess so gesteuert, dass durch Dehydratation Dimethylether als Hauptprodukt anfällt. Der Dimethylether (DME) wird nachfolgend durch weitere Dehydratation in ein wasserhaltiges Kohlenwasserstoffgemisch umgewandelt. Dieses Kohlenwasserstoffgemisch hat beispielsweise folgende Zusammensetzung:
- 57 % Wasser
- 5 % Propan
- 38 % Kohlenwasserstoffe (hauptsächlich im Bereich C4 bis C12).

Die Kohlenwasserstoffe bestehen aus Paraffinen, Olefinen, Naphthenen und Aromaten. Das unmittelbar am Förderort hergestellte Kohlenwasserstoffgemisch wird nunmehr entweder unbehandelt oder nach Entgasung und/oder Entwässerung dem schweren Rohöl zugesetzt, wobei dieses verdünnt und dadurch die Transportfähigkeit deutlich verbessert wird.

Über die Zusatzmenge an Kohlenwasserstoffgemisch lässt sich die Viskosität entsprechend der gewünschten Transportqualität gezielt einstellen. Zur Herabsetzung der Viskosität des schweren Rohöls werden in Abhängigkeit vom API-Grad bis zu 40 %, bezogen auf die Menge an schwerem Rohöl, zugesetzt. Dadurch wird eine für einen Transport ausreichend hohe Verdünnung erzielt.

Es können auch größere Mengen zugesetzt werden, die sich jedoch nur noch unwesentlich auf eine weitere Verringerung der Viskosität auswirken. Bereits kleine Zusatzmengen, im einstelligen Prozentbereich, können ausreichen, um die Qualität des schweren Rohöls zu verbessern. Vorzugsweise führen mindestens 10 %, bezogen auf die Menge an ungereinigtem Rohöl, zu sehr guten Ergebnissen.

Von großem ökonomischem Vorteil ist einerseits die Verwertung des als Nebenprodukt anfallenden Erd- bzw. Begleitgases unmittelbar am Entstehungsort und andererseits die Tatsache, dass das zu fließfähigerem und transportfähigem Rohöl modifizierte schwere Rohöl keiner gesonderten Behandlung unterzogen werden muss. Es kann nunmehr in einer Raffinerie wie normales leichtes Rohöl weiterverarbeitet werden. Unter "leichtem Rohöl" sind dabei solche Rohöle zu verstehen, die einen API von in etwa 30° oder größer besitzen.

Als Nebeneffekt lassen sich mit leichtem Erdöl höhere Verkaufserlöse erzielen als mit schwerem Erdöl.

Dadurch amortisiert sich der Aufwand für die Errichtung einer Anlage zur chemischen Umsetzung von Erdgas bzw. Erdölbegleitgas in Kohlenwasserstoffgemisch bereits nach relativ kurzer Betriebsdauer.

Dem schweren Rohöl wird ein Kohlenwasserstoffgemisch vorzugsweise mit einer Kettenlänge von C4 bis C12 zugesetzt.

Die speziellen Bedingungen zur Gewinnung des wasserhaltigen Kohlenwasserstoffgemisches sind im nachfolgenden Ausführungsbeispiel angegeben.

Das als Zwischenprodukt gebildete Methanol sollte vorzugweise noch einen Restwassergehalt von 4 % besitzen und wird durch Dehydratation katalytisch zu einem wasser- und gashaltigen Kohlenwasserstoffgemisch umgesetzt.

Dieses Kohlenwasserstoffgemisch kann unmittelbar im Bereich der Erdöllagerstätte zur Verbesserung der Förderfähigkeit entweder dem bereits geförderten und/oder dem noch unterirdisch gelagerten schweren Rohöl über das Bohrloch zugeführt werden. Vorzugsweise erfolgt die Einbringung in ein Bohrloch über ein in dieses eingesetztes Spülrohr.

Bei einzelnen Schächten zur Förderung eines Erdöl-Clusters besteht auch die Möglichkeit, dass eine erste Teilmenge an Kohlenwasserstoffgemisch oberhalb des Bohrloches zugeführt wird, um die Transportfähigkeit des schweren Rohöls zu verbessern. Die Erdölschächte eines Clusters werden zusammengeführt, wobei nach dem Zusammenführen die Fluidströme vermischt und in einer Massenabtrenneinrichtung Wasser und Ölbegleitgas abgetrennt werden. Während des Vermischens kann wiederum eine entsprechende Menge an Kohlenwasserstoffgemisch zugesetzt werden. Dieses wird in Abhängigkeit von der Viskosität des Schweröls so dosiert, dass dessen Transportfähigkeit in ausreichendem Maße verbessert wird bis zu einer Qualität, wie leichtes Rohöl.

Erforderlichenfalls kann das gebildete Kohlenwasserstoffgemisch vor dem Inkontaktbringen mit dem schweren Rohöl noch gereinigt, also entwässert und entgast werden. Durch separate Wasserabtrennung und Entgasung kann aus dem wässrigen Kohlenwasserstoffgemisch ein wasserfreies Kohlenwasserstoffgemisch erzeugt werden.

Grundsätzlich kann das aufbereitete Kohlenwasserstoffgemisch an jeder gewünschten Stelle zur Verbesserung der Förder- oder Transportfähigkeit zugesetzt werden. Gereinigtes Kohlenwasserstoffgemisch kann vorzugsweise auf der Saugseite der zum Transport des Rohöls eingesetzten Pumpe zugeführt werden. Gegebenenfalls können Kohlenwasserstoffgemisch und schweres Rohöl auch in einer separaten Mischeinrichtung zu leichtem Rohöl vermischt werden.

Einsatzmengen an Kohlenwasserstoffgemisch von ca. 20 % sind bereits ausreichend, um z.B. Schweröl (API 23°) in leichtes Rohöl (API 31°) umzuwandeln.

Vorzugsweise wird die Viskosität des geförderten schweren Rohöls gemessen und in Abhängigkeit vom aktuellen Messergebnis die Menge an Kohlenwasserstoffgemisch dosiert zugesetzt, um leichtes Rohöl zu erhalten.

Unaufbereitetes Kohlenwasserstoffgemisch muss innerhalb der Förder- und Transportstrecke des schweren Rohöls diesem vor Erreichen der Massenabtrenneinrichtung zugesetzt werden. Dagegen kann aufbereitetes Kohlenwasserstoffgemisch, das entwässert und entgast ist, an allen Stellen der Förder- und Transportstrecke dem schweren Rohöl zugesetzt werden.

Die Erfindung wird nachstehend an einem Beispiel näher erläutert.

In der zugehörigen Zeichnung ist die Verfahrensweise in einem Fließschema dargestellt. In einer Erdöllagerstätte werden 1088 t/h an schwerem Rohöl (API 23°) gefördert, das folgende Zusammensetzung aufweist:

| | |
|---|---|
| Kohlenwasserstoffe | 818 t |
| Wasser | 240 t und |
| Gasförmige Bestandteile | 30 t. |

In einer zentralen Ölaufarbeitung 1 wird das aus unterschiedlichen Bohrlöchern 2 stammende Rohöl zusammengeführt, gemischt und nachfolgend einer Trenneinrichtung zugeführt, in der die wässrige Phase und gasförmige Bestandteile abgetrennt werden. Die Trenneinrichtung ist Bestandteil der zentralen Ölaufarbeitung 1. In dem Fließschema sind symbolhaft drei Bohrlöcher 2 dargestellt.

Im Zusammenhang mit der Erdölförderung fällt Erdgas/Erdölbegleitgas mit folgender Zusammensetzung an:

| | |
|---|---|
| - Stickstoff | 1,5 % |
| - Methan | 92 % |
| - Ethan | 3,5% |
| - Propan | 1,5% |
| - höhere Kohlenwasserstoffe | 1 % |
| - Schwefel | 50 ppm. |

Das Erdgas/Ölbegleitgas (350.000 Nm³/h) wird in einer auf dem Gelände der Erdöllagerstätte errichteten Chemieanlage wie folgt in ein Kohlenwasserstoffgemisch umgewandelt. Das Erdgas/Ölbegleitgas 3 wird bei einem Druck von 7 MPa (70 bar) zunächst bei einer Temperatur von 375 °C über einem Zinkoxidbett entschwefelt (Entschwefelung 4), danach mit Prozesskondensat und Dampf gesättigt (Sättiger 5) und nach Einstellung eines Dampf/Kohlenstoffverhältnisses von 1,0 im Prereformer 6, einem adiabatisch arbeitenden Katalysereaktor, bei 480 °C in ein Gemisch aus Methan, Kohlendioxid und Kohlenmonoxid vorgespalten.

Nach weiterer Aufheizung auf 630 bis 650 °C wird das vorgespaltene Gas einem Autothermreformer 7 zugeführt. In diesem Katalysereaktor wird durch Zusatz von auf 230 °C vorgeheiztem Sauerstoff 9, der in einer Luftzerlegungsanlage 8 gewonnen wird, bei 1030 °C ein Synthesegas 10 erzeugt, das aus Wasserstoff, Kohlenmonoxid und Kohlendioxid besteht und nur noch eine sehr kleine Menge ungespaltenes Methan enthält. Dieses Synthesegas wird in einem Abhitzesystem 11 gekühlt.

Über verschiedene Stufen, die zur Dampferzeugung bzw. Erwärmung verschiedener Gas-/Produktströme genutzt werden, wird das nunmehr bei 5,5 MPa (55 bar) anliegende und gekühlte Synthesegas mit einem Kompressor 12 auf 7,5 MPa (75 bar) komprimiert. Nachfolgend wird in einem dualen System, bestehend aus einem wassergekühlten und einem gasgekühlten Reaktor 13, Synthesegas katalytisch im Temperaturbereich von 220 bis 260 °C in Methanol umgewandelt und durch Kondensation ein Rohmethanol 14 mit folgender Zusammensetzung erhalten:

| | |
|---|---|
| - Methanol | 83 Gew.-% |
| - Kohlendioxid | 3,6 Gew.-% |
| - Wasser | 11,7 Gew.-% |
| - Methan | 1,5 Gew.-% |
| - höhere Kohlenwasserstoffe | 0,1 Gew.-% |
| - höhere Alkohole | 0,1 %. |

Während der Methanolsynthese wird eine Teilmenge an Synthesegas im Kreislauf 15 gefahren und dabei mittels eines weiteren Kompressors 16 auf den erforderlichen Druck gebracht. Aufgrund der im Synthesegas enthaltenen Verunreinigungen wird eine Teilmenge an Synthesegas als Purgegas 17 ausgekreist und über eine Pressure-Swing-Anlage (PSA) 18 gefahren. Dieser wird auch unter hohem Druck ein Synthesegasteilstrom 19 zugeführt, der nach der Druckerhöhung mittels des Kompressors 12 abgezweigt wird. Der in der PSA 18 erzeugte Wasserstoff 20 wird auf der Saugseite des Synthesegaskompressors 12 in den Synthesegasstrom 10 zurückgeführt.

Das nach der Methanolsynthese 13 in mehreren Stufen kondensierte Rohmethanol 14 wird in einer nachgeschalteten Destillationsanlage 21 zunächst entgast und nachfolgend von niedrig siedenden sowie abschließend höher siedenden Produkten gereinigt. Gegenüber der klassischen dreistufigen Destillation zur Herstellung von marktfähigem Methanol wird die Destillation im Temperaturbereich von 70 bis 140 °C in nur zwei Kolonnen ausgeführt und ein Restwassergehalt im erzeugten Methanol von 4 % eingestellt. Insgesamt fallen nach der Destillation 435 t/h Rohmethanol an, die 17 t Wasser enthalten.

Das auf 4 % Wassergehalt destillierte Methanol wird nachfolgend in einem Festbettreaktor 22 (DME-Reaktor) katalytisch in ein DME(Dimethylether)/Methanol/Wasser-Gemisch umgewandelt. Das Reaktionsprodukt aus dem DME-Reaktor wird mit Recyclegas 23 zur Temperatureinstellung versetzt und nachfolgend in weiteren adiabatisch arbeitenden Reaktoren 24 im Temperaturbereich von 320 bis 420 °C in ein Kohlenwasserstoff/Wasser-Gemisch umgewandelt. Aus den eingesetzten 435 t/h Methanol entstehen dabei 191 t Kohlenwasserstoffe und 244 t Wasser. Dieses wässrige Kohlenwasserstoffgemisch wird abschließend in einer Entgasungseinheit 25 entgast und dem unaufbereiteten schweren Rohöl zugesetzt.

Gemäß diesem Beispiel werden dem unaufbereiteten schweren Rohöl (1088 t/h) kontinuierlich 4351 wässriges Kohlenwasserstoffgemisch pro Stunde zugemischt. Im Fließschema ist die Stelle des Zumischens mit dem Bezugszeichen 26 gekennzeichnet.

Die Zumischung des wässrigen Kohlenwasserstoffgemisches erfolgt vor dem Trennprozess Rohöl/Erdölbegleitgas, der innerhalb der zentralen Ölaufbereitung 1 stattfindet. Nachfolgend werden in der zentralen Ölaufbereitung 1 aus dem verdünnten Rohölgemisch die wässrige Phase und noch vorhandene gasförmige Bestandteile, wie Stickstoff, Kohlendioxid, Methan und Ethan, abgetrennt. Es werden 1004 t/h aufbereitetes Rohöl mit einem API 36° erhalten. Dieses kann nunmehr in herkömmlichen Transportrohrleitungen 27 mit Pumpstationen über tausende von Kilometern ohne Probleme transportiert werden. Dieses modifizierte Rohöl besitzt eine Qualität wie leichtes Rohöl.

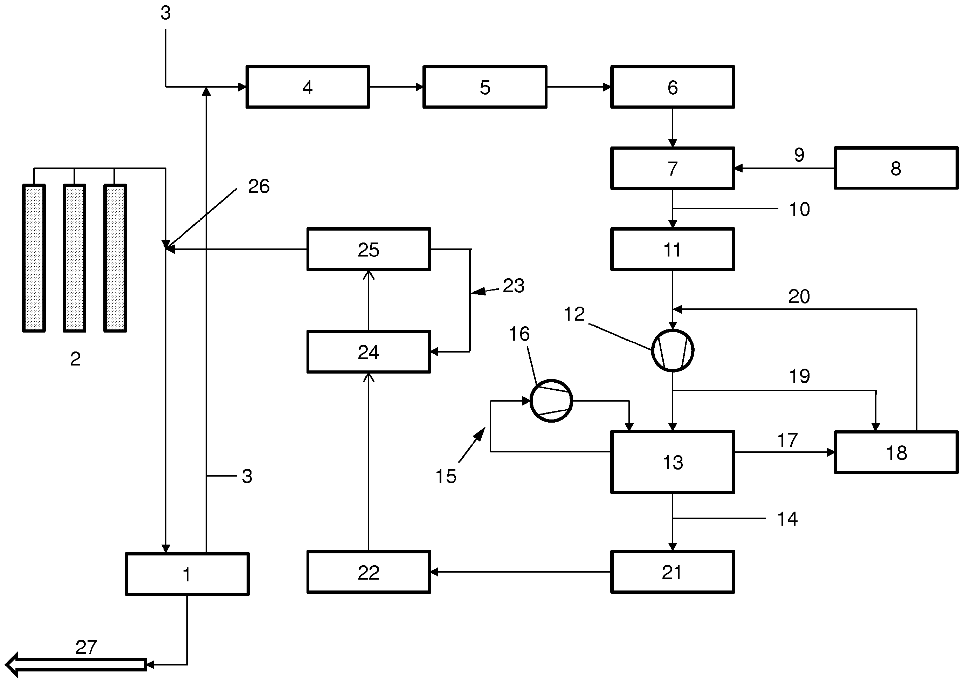

## Patentansprüche

1. Verfahren zur Verbesserung der Transportfähigkeit von schwerem Rohöl, wobei im Bereich einer Erdöllagerstätte anfallendes Erdgas und/oder Erdölbegleitgas in ein Methanol-Wasser-Gemisch umgewandelt wird, dieses in Dimethylether und dieser nachfolgend in ein wasserhaltiges Kohlenwasserstoffgemisch, das dem schweren Rohöl zugesetzt wird.

2. Verfahren nach Anspruch 1, **dadurch gekennzeichnet, dass** die Umwandlung des Erd- und/oder Erdölbegleitgases in ein Methanol-Wasser-Gemisch mittels folgender Verfahrensschritte vorgenommen wird: Entschwefelung; Vorspaltung in ein Gasgemisch aus Methan, Kohlendioxid und Kohlenmonoxid; katalytische Umwandlung des vorgespalteten Gasgemisches unter erhöhter Temperatur und bei einem Druck von mindestens 5 MPa (50 bar) in einem Autothermreaktor in Synthesegas unter Zusatz von vorgeheiztem Sauerstoff; nachfolgender Komprimierung und katalytischer Umwandlung des Synthesegases im Rahmen einer zweistufigen Wasser-Methanol-Synthese in einem wassergekühlten und in einem gasgekühlten Reaktor in Methanol, wobei durch nachfolgende mehrstufige Kondensation Rohmethanol (Methanol-Wasser-Gemisch) erhalten wird.

3. Verfahren nach einem der Ansprüche 1 oder 2, **dadurch gekennzeichnet, dass** das erhaltene Methanol-Wasser-Gemisch (Rohmethanol) einer zweitstufigen Destillation unterzogen wird, wobei in der ersten Stufe niedrig siedende und in der zweiten Stufe höher siedende Verbindungen abgetrennt werden und ein Destillat mit einem hohen Wasser- und Alkoholgehalt entsteht, das nachfolgend in einem Festbettreaktor katalytisch in ein Dimethylether/Methanol/Wasser-Gemisch umgewandelt wird, das anschließend in weiteren adiabatisch arbeitenden Reaktoren im Temperaturbereich von 300 bis 450 °C in ein Kohlenwasserstoff/Wasser-Gemisch als Endprodukt umgewandelt wird.

4. Verfahren nach einem der Ansprüche 1 bis 3, **dadurch gekennzeichnet, dass** das im Festbettreaktor anfallende Dimethylether/Methanol/Wasser-Gemisch mit Recyclegas zur Temperatureinstellung versetzt wird.

5. Verfahren nach einem der Ansprüche 1 bis 4, **dadurch gekennzeichnet, dass** das gebildete Kohlenwasserstoffgemisch unmittelbar, im Bereich der Erdöllagerstätte, entweder dem bereits geförderten und/oder dem noch unterirdisch gelagerten schweren Rohöl über das Bohrloch zugeführt wird.

6. Verfahren nach einem der Ansprüche 1 bis 5, **dadurch gekennzeichnet, dass** das gebildete Kohlenwasserstoffgemisch über ein Spülrohr in das Bohrloch eingebracht wird.

7. Verfahren nach einem der Ansprüche 1 bis 6, **dadurch gekennzeichnet, dass** das gebildete Kohlenwasserstoffgemisch vor dem Inkontaktbringen mit dem schweren Rohöl entwässert und entgast wird.

8. Verfahren nach einem der Ansprüche 1 bis 7, **dadurch gekennzeichnet, dass** aufbereitetes Kohlenwasserstoffgemisch, das entwässert und entgast ist, vor oder nach der zentralen Ölaufarbeitung dem schweren Rohöl zugesetzt wird.

9. Verfahren nach einem der Ansprüche 1 bis 7, **dadurch gekennzeichnet, dass** nicht aufbereitetes Kohlenwasserstoffgemisch vor der zentralen Ölaufarbeitung dem schweren Rohöl zu gesetzt wird.

10. Verfahren nach einem der Ansprüche 1 bis 9, **dadurch gekennzeichnet, dass** nach der Abtrennung von Wasser und Ölbegleitgas aus dem Rohöl diesem eine weitere Menge an aufbereitetem Kohlen Wasserstoffgemisch zugesetzt wird, die in Abhängigkeit von der Viskosität des Schweröls so dosiert wird, dass ein leichtes Rohöl gebildet wird.

11. Verfahren nach einem der Ansprüche 1 bis 10, **dadurch gekennzeichnet, dass** aufbereitetes Kohlenwasserstoffgemisch auf der Saugseite der zum Transport des Rohöls eingesetzten Pumpe zugeführt wird.

12. Verfahren nach einem der Ansprüche 1 bis 11, **dadurch gekennzeichnet, dass** die Viskosität des geförderten schweren Rohöls gemessen und in Abhängigkeit vom aktuellen Messergebnis die Menge an Kohlenwasserstoffgemisch dosiert zugesetzt wird, um leichtes Rohöl zu erhalten.

13. Verfahren nach einem der Ansprüche 1 bis 12, **dadurch gekennzeichnet, dass** aufbereitetes Kohlenwasserstoffgemisch und schweres Rohöl in einer separaten Mischeinrichtung vermischt werden.

## Claims

1. A method for improving the transportability of heavy crude oil, wherein natural gas and/or mineral oil-associated gas produced in the area of a mineral oil deposit is converted into a methanol/water mixture, that is converted into dimethyl ether, and that subsequently into a water-containing hydrocarbon mixture, which is added to the heavy crude oil.

2. The method according to claim 1, **characterized in that** the conversion of the natural and/or mineral oil-associated gas into a methanol-water mixture is carried out by means of the following method steps: desulfurization; pre-cracking into a gas mixture of methane, carbon dioxide and carbon monoxide; catalytic conversion of the pre-cracked gas mixture into synthesis gas at elevated temperature and a pressure of at least 5 MPa (50 bar) in an autothermal reactor while adding preheated oxygen; subsequent compression and catalytic conversion of the synthesis gas into methanol in a two-stage water-methanol synthesis in a water-cooled and in a gas-cooled reactor, wherein crude methanol (methanol-water mixture) is obtained by a subsequent multi-stage condensation.

3. The method according to any one of the claims 1 to 2, **characterized in that** the methanol/water mixture (crude methanol) obtained is subjected to a two-stage distillation, wherein, in the first stage, low-boiling compounds are separated off, and in the second stage higher-boiling compounds are separated off, and a distillate having a high water and alcohol content is formed, which is then catalytically converted in a fixed-bed reactor into a dimethyl ether/methanol/water mixture, which is then converted in further adiabatically operating reactors in the temperature range from 300 to 450°C into a hydrocarbon/water mixture as an end product.

4. The method according to any one of the claims 1 to 3, **characterized in that** the dimethyl ether/methanol/water mixture arising in the fixed-bed reactor is admixed with recycled gas for temperature adjustment.

5. The method according to any one of the claims 1 to 4, **characterized in that** the hydrocarbon mixture formed is fed directly, in the area of the mineral oil deposit, either to the heavy crude oil already extracted and/or via the borehole to the heavy crude oil still stored underground.

6. The method according to any one of the claims 1 to 5, **characterized in that** the hydrocarbon mixture formed is introduced into the borehole via a purge tube.

7. The method according to any one of the claims 1 to 6, **characterized in that** the hydrocarbon mixture formed is dewatered and degassed before it is brought into contact with the heavy crude oil.

8. The method according to any one of the claims 1 to 7, **characterized in that** treated hydrocarbon mixture that is dewatered and degassed is added to the heavy crude oil before or after the central oil processing.

9. The method according to any one of the claims 1 to 7, **characterized in that** non-treated hydrocarbon mixture is added to the heavy crude oil before the central oil processing.

10. The method according to any one of the claims 1 to 9, **characterized in that**, after separating off water and oil-associated gas from the crude oil, a further amount of treated hydrocarbon mixture is added thereto, which, depending on the viscosity of the heavy oil, is metered in such a manner that a light crude oil is formed.

11. The method according to any one of the claims 1 to 10, **characterized in that** treated hydrocarbon mixture is supplied on the suction side of the pump used for the transport of the crude oil.

12. The method according to any one of the claims 1 to 11, **characterized in that** the viscosity of the extracted heavy crude oil is measured and the quantity of hydrocarbon mixture is added in a metered manner, depending on the current measurement result, in order to obtain light crude oil.

13. The method according to any one of the claims 1 to 12, **characterized in that** treated hydrocarbon mixture and heavy crude oil are mixed in a separate mixing device.

## Revendications

1. Procédé d'amélioration de l'aptitude à l'écoulement de pétrole brut lourd, dans lequel du gaz naturel et/ou du gaz associé au pétrole résultant au niveau d'un gisement pétrolifère est transformé en un mélange méthanol/eau, ce mélange est transformé en diméthyléther et ce dernier est, à son tour, transformé en un mélange d'hydrocarbures aqueux qui est ajouté au pétrole brut lourd.

2. Procédé selon la revendication 1, **caractérisé par le fait que** la transformation du gaz naturel et/ou du gaz associé au pétrole en un mélange méthanol/eau est effectuée au moyen des étapes de procédé suivantes: une désulfuration; une pré-décomposition en un mélange gazeux de méthane, de dioxyde de carbone et de monoxyde de carbone; une transformation catalytique du mélange gazeux pré-décomposé à température élevée et à une pression d'au moins 5 MPa (50 bars), dans un réacteur autothermique, en gaz de synthèse avec addition d'oxygène préchauffé; une compression et une transformation catalytique ultérieures du gaz de synthèse dans le cadre d'une synthèse eau-méthanol à deux étapes, dans un réacteur refroidi à l'eau et dans un réacteur refroidi au gaz, en méthanol, du méthanol brut (mélange méthanol-eau) étant obtenu par une condensation à plusieurs étages qui a lieu ensuite.

3. Procédé selon l'une quelconque des revendications 1 ou 2, **caractérisé par le fait que** le mélange méthanol/eau obtenu (méthanol brut) est soumis à une distillation à deux étapes, dans lequel des composés à bas point d'ébullition sont séparés dans la première étape et des composés à point d'ébullition plus haut sont séparés dans la deuxième étape et un distillat ayant une haute teneur en eau et en alcool est créé qui est ensuite transformé de façon catalytique, dans un réacteur à lit fixe, en un mélange diméthyléther/méthanol/eau qui est ensuite transformé, dans d'autres réacteurs travaillant de façon adiabatiques, dans la plage de températures comprise entre 300 et 450 °C, en un mélange d'hydrocarbures/eau en tant que produit final.

4. Procédé selon l'une quelconque des revendications 1 à 3, **caractérisé par le fait que** le mélange de diméthyléther/méthanol/eau obtenu dans le réacteur à lit fixe est mélangé avec du gaz de recyclage pour le réglage de la température.

5. Procédé selon l'une quelconque des revendications 1 à 4, **caractérisé par le fait que** le mélange d'hydrocarbures formé est amené directement, au niveau du gisement pétrolifère, soit au pétrole brut lourd déjà extrait et/ou au pétrole brut lourd encore stocké sous terre, via le forage.

6. Procédé selon l'une quelconque des revendications 1 à 5, **caractérisé par le fait que** le mélange d'hydrocarbures formé est introduit dans le trou de forage via un tuyau de rinçage.

7. Procédé selon l'une quelconque des revendications 1 à 6, **caractérisé par le fait que** le mélange d'hydrocarbures formé est déshydraté et dégazé avant d'être mis en contact avec le pétrole brut lourd.

8. Procédé selon l'une quelconque des revendications 1 à 7, **caractérisé par le fait que** du mélange d'hydrocarbures traité qui est déshydraté et dégazé est ajouté au pétrole brut lourd avant ou après le traitement central du pétrole.

9. Procédé selon l'une quelconque des revendications 1 à 7, **caractérisé par le fait que** du mélange d'hydrocarbures non traité est ajouté au pétrole brut lourd avant le traitement central du pétrole.

10. Procédé selon l'une quelconque des revendications 1 à 9, **caractérisé par le fait qu'**après avoir séparé l'eau et le gaz associé au pétrole du pétrole brut, on ajoute à ce dernier une autre quantité de mélange d'hydrocarbures traité qui est dosée en fonction de la viscosité du pétrole lourd de manière à ce qu'un pétrole brut léger soit formé.

11. Procédé selon l'une quelconque des revendications 1 à 10, **caractérisé par le fait que** du mélange d'hydrocarbures traité est amené du côté aspiration de la pompe utilisée pour transporter le pétrole brut.

12. Procédé selon l'une quelconque des revendications 1 à 11, **caractérisé par le fait que** la viscosité du pétrole brut lourd extrait est mesurée et que, en fonction du résultat de mesure actuel, la quantité de mélange d'hydrocarbures est ajoutée de manière dosée pour obtenir du pétrole brut léger.

13. Procédé selon l'une quelconque des revendications 1 à 12, **caractérisé par le fait que** du mélange d'hydrocarbures traité et du pétrole brut lourd sont mélangés entre eux dans un dispositif de mélange séparé.
